# EUROPÄISCHE PATENTSCHRIFT

(11) **EP 0 449 919 B1**
(45) Veröffentlichungstag und Bekanntmachung des Hinweises auf die Patenterteilung: **04.05.1994**
(21) Anmeldenummer: 90900865.8
(22) Anmeldetag: 15.12.1989
(51) Int. Cl.: C07C 69/54, C07C 67/08

(54) **VERFAHREN ZUR VERBESSERTEN HERSTELLUNG VON (METH)ACRYLSÄUREESTERN MEHRWERTIGER ALKOHOLE (IV)**
IMPROVED PROCESS FOR PRODUCING ESTERS OF (METH)ACRYLIC ACID AND POLYHYDRIC ALCOHOLS (IV)
PROCEDE AMELIORE DE PRODUCTION D'ESTERS D'ACIDE (METH)ACRYLIQUE D'ALCOOLS POLYVALENTS (IV)

(30) Priorität: 24.12.1988 DE 3843843
(43) Veröffentlichungstag der Anmeldung: 09.10.1991
(73) Patentinhaber: Henkel Kommanditgesellschaft auf Aktien, 40191 Düsseldorf (DE)
(72) Erfinder: RITTER, Wolfgang, D-5657 Haan (DE); SITZ, Hans-Dieter, D-4049 Rommerskirchen (DE); SPEITKAMP, Ludwig, D-4000 Düsseldorf 13 (DE)
(74) Vertreter: von Kreisler, Alek, Dipl.-Chem.
(86) Internationale Anmeldenummer: EP8901546
(87) Internationale Veröffentlichungsnummer: WO9007483

(56) Entgegenhaltungen:
- US-A- 3 988 213

## Beschreibung

Die Erfindung betrifft ein Verfahren zur Herstellung von Estern der Acrylsäure und/oder der Methacrylsäure - im folgenden als (Meth)acrylsäureester bezeichnet - mit mehrwertigen Alkoholen durch Umsetzung der Reaktanten in Gegenwart saurer Veresterungskatalysatorer, unter Zusatz von Polymerisationsinhibitoren zum Reaktionsgemisch. Das eriindungsgemäße Verfahren arbeitet insbesondere mit der zusätzlichen Maßnahme, den Reaktionsraum mit einem Sauerstoff enthaltenden Gasstrom zu durchspülen, um die Inhibitorwirkung zu verbessern und den Austrag des als Kondensationsprodukt aus der Veresterung anfallenden Wassers aus dem Reaktionsraum zu fördern.

(Meth)acrylsäureester mehrwertiger Alkohole insbesondere aus der Gruppe der 2- bis 4wertigen aliphatischen gesättigten Alkohole und deren Oxalkylierungsprodukte finden zunehmende Bedeutung als hochreaktive Bestandteile in strahlenhärtenden Systemen. Solche polyfunktionellen (Meth)acrylsäureester können beispielsweise als Lackrohstoffe für die Elektronenstrahlhärtung oder als Bestandteil von UV-Licht-härtenden Druckfarben oder entsprechenden Überzugslacken, Spachtel, Form- oder Vergußmassen oder auch in Klebstoffen insbesondere anaerob härtenden Klebstoffen Verwendung finden. Ihre Herstellung ist allerdings nicht problemlos. Gefordert werden insbesondere farblose Produkte mit geringer Säurezahl, hoher Lagerstabilität, die praktisch auch keinen Eigengeruch aufweisen. Eine destillative Reinigung der (Meth)acrylsäureester der hier betroffenen Art scheidet in aller Regel aufgrund ihres hohen Molekulargewichtes und ihrer hohen Reaktivität aus. Die Produkte sollen also unmittelbar als möglichst farblose Reaktionsprodukte der Veresterung anfallen. Die Durchführung der Veresterungsreaktion fordert die Mitverwendung hochwirksamer Inhibitoren, die ihrerseits keine unerwünschten Nebenreaktionen beispielsweise Verfärbungen auslösen. Es kann weiterhin wünschenswert sein, bei der Veresterung nicht etwa nur das flüssige Reaktionsprodukt vor unerwünschten Polymerisationsreaktionen zu schützen, sondern auch eine hinreichende Inhibierung des gesamten Reaktionsraumes, und zwar sowohl des Gasinnenraumes wie der Wandflächen, die mit diesem Gasinnenraum in Kontakt stehen, sicherzustellen. Begegnet wird damit der Gefahr unerwünschter Polymerisatbildung beispielsweise an solchen nicht geschützten Wandbereichen, wobei das Abwaschen solcher Polymerisate in das Reaktionsprodukt wiederum zur unerwünschten Viskositätssteigerung im Endprodukt bzw. zu unerwünschten unlöslichen Partikeln führt.

Zur Herstellung solcher polyfunktioneller (Meth)acrylsäureester mehrwertiger Alkohole besteht umfangreiche Vorliteratur. Verwiesen sei insbesondere auf die deutsche Offenlegungsschrift 29 13 218 und die darin zitierte einschlägige Literatur. So ist es aus der DE-AS 12 67 547 und aus der Zeitschrift "Chem. and Ind." 18 (1970), 597, bekannt, polyfunktionelle (Meth)acrylsäureester durch azeotrope Veresterung der (Meth)acrylsäure mit mehrwertigen Alkoholen in Gegenwart von sauren Katalysatoren und Polymerisationsinhibitoren wie Phenolen, Phenolderivaten, Kupfer, Kupferverbindungen oder Phenothiazin herzustellen. Als saure Katalysatoren werden organische oder anorganische Säuren oder saure Ionenaustauscher eingesetzt, wobei p-Toluolsulfonsäure und Schwefelsäure bevorzugt sein können. Die Veresterung erfolgt unter azeotropen Bedlingungen unter Mitverwendung von Schleppmitteln für die Entfernung des Reaktionswassers beispielsweise bei Temperaturen von 40 bis 120 ^{o}C. Geeignete Schleppmittel sind etwa aliphatische oder cycloaliphatische oder aromatische Kohlenwasserstoffe bzw. deren Gemische mit Siedebereichen innerhalb der angegebenen Temperaturgrenzen.

In der genannten DE-OS 29 13 218 wird zur Verbesserung dieser Veresterungsreaktion und zur Gewinnung von nicht durch Destillation gereinigten (Meth)acrylsäureestern der genannten Art vorgeschlagen, die azeotrope Veresterung in Gegenwart mindestens eines organischen Esters der phosphorigen Säure zusätzlich zu einem mitverwendeten Inhibitor auf Phenolbasis durchzuführen. Dieser Inhibitor auf Phosphitbasis soll insbesondere mit Hilfe eines Trägergases zudosiert werden und dient ersichtlich vor allem dazu, eine Inhibierung des mit Gas- bzw. Dampfphase erfüllten Reaktionsinnenraumes zu fördern und die Farbintensität zu reduzieren. So ist dann insbesondere auch vorgesehen, nach der azeotropen Veresterung Restlösungsmittel, Reste von Reaktivbestandteilen und flüchtige Nebenprodukte durch Einblasen von Luft oder Stickstoff aus dem Veresterungsprodukt zu entfernen, wobei diese Gasphase mit organischen Phosphiten angereichert oder gesättigt sein soll.

Die Erfindung geht von dieser Problemstellung aus, nicht etwa nur die reaktive Flüssigphase durch Polymerisationsinhibitoren wirkungsvoll zu stabilisieren, sondern jetzt zusätzlich auch den gesamten Reaktorinnenraum vor unerwünschten Polymerisationsauslösungen zu schützen. Dabei will die Erfindung aber auf die Mitverwendung gasförmiger komplexer Inhibitorsysteme verzichten. Aufgabe der Erfindung ist es insbesondere, den gleichen Inhibitor sowohl für den Schutz der reaktiven Flüssigphase als auch für den Schutz des Gas- bzw. Dampf-erfüllten Innenraumes und der mit diesen Anteilen des Reaktionsinnenraumes in Kontakt stehenden Feststofflächen eirzusetzen. Gemäß einer weiteren Aufgabe der Erfindung soll es möglich sein, den im praktischen Einsatz gewünschten Applikationsinhibitor der hier betroffenen hochreaktiven Systeme gleichzeitig schon als Reaktionsinhibitor bei der Synthese der polyfunktionellen (Meth)acrylsäureester einzusetzen. Die erfindungsgemäßen Aufgabenstellungen werden durch die neue im nachfolgenden geschilderte Verfahrenstechnologie gelöst.

Gegenstand der Erfindung ist dementsprechend ein Verfahren zur Herstellung von (Meth)acrylsäureestern mehrwertiger Alkohole durch Umsetzung der Reaktanten in Gegenwart von sauren Veresterungskatalysatoren unter Zusatz von Polymerisationsinhibitoren zum Reaktionsgemisch und Durchspülen des Reaktionsraumes mit einem Sauerstoff enthaltenden Gasstrom, wobei das neue Verfahren dadurch gekennzeichnet ist, daß man den mit Gasphase erfüllten Anteil des Reaktionsinnenraumes mit fein verteilten Flüssigkeitströpfchen belädt, die Polymerisationsinhibitor enthalten.

In der bevorzugten Ausführungsform der Erfindung wird die Inhibitor enthaltende Flüssigphase dem die Gasphase enthaltenden Reaktionsinnenraum in fein-disperser Form in solcher Menge zugeführt, daß alle von der Gasphase berührten Festkörperinnenflächen mit einem bevorzugt geschlossenen Inhibitor enthaltenden Flüssigkeitsfilm benetzt sind. Es ist dabei weiterhin bevorzugt, das Einbringen der Inhibitor enthaltenden Flüssigphase in den die Gasphase enthaltenden Reaktionsinnenraum ohne Ausbildung wesentlicher Schattenräume vorzunehmen - mit anderen Worten dafür Sorge zu tragen, daß tatsächlich auch der gesamte Gasinnenraum hinreichend mit feinverteilten Flüssigkeitströpfchen beladen ist. In einer technisch besonders wichtigen Ausführungsform wird die Inhibitor enthaltende Flüssigphase durch Versprühen in den fein-dispersen Zustand gebracht.

Der neue technologische Ansatz der erfindungsgemäßen Lehre stellt sich damit wie folgt dar: Anstelle der Inhibierung des gas- bzw. dampferfüllten Innenraumes und der damit in Kontakt stehenden Wandinnenflächen mit gasförmig eingebrachten Inhibitoren wird eine zunächst feindisperse Flüssigphase im Gasraum vorgesehen, die Inhibitor enthält. An den mit der Gasphase in Kontakt stehenden Wandflächen kondensieren die Flüssigkeitströpfchen. Die Menge der absatzweise oder bevorzugt kontinuierlich in den Gasinnenraum eingebrachten und Inhibitor enthaltenden dispersen Flüssigphase wird dabei insbesondere so bemessen, daß sich an den Wandinnenflächen ein zusammenhängender Flüssigkeitsfilm ausbilden kann, der seinerseits Polymerisationsinhibitor enthält. In der angegebenen Weise sind alle nur denkbaren Gefahrenstellen für die unerwünschte Polymerisationsauslösung mit Inhibitor enthaltender Flüssigphase geschützt, so daß die optimale Inhibierung des Reaktionsansatzes während der gesamten Reaktionsphase sichergestellt ist.

In einer besonders wichtigen Ausführungsform wird dieses neue Prinzip wie folgt verwirklicht: Anteile des flüssigen, den Polymerisationsinhibitor enthaltenden Reaktionsgemisches werden absatzweise oder kontinuierlich feinstteilig in den Reaktionsinnenraum, und zwar insbesondere in den mit Gas- bzw. Dampfphase erfüllten Reaktionsinnenraum eingetragen, so daß sich dieser Bereich mit der feinstverteilten und Inhibitor enthaltenden Flüssigphase belädt. Die Menge des so eingebrachten Reaktionsgutes wird dabei so bemessen, daß sich an den Reaktionsinnenwänden geschlossene Flüssigkeitsfilme ausbilden und im Verlauf der Reaktion an diesen Wänden nach unten in den Reaktionssumpf ablaufen und sich damit mit der Hauptmenge des Reaktionsgutes wieder vereinigen. Die Vorteile dieser Verfahrensweise sind einleuchtend. Absatzweise oder kontinuierlich wird immer wieder frische, Inhibitor enthaltende feinstverteilte Flüssigphase in den Gasinnenraum dosiert. Diese disperse Phase kann durch Tröpfchenvereinigung insbesondere aber auch durch Kondensation an den Reaktorinnenwänden zusammenfließen und dem Reaktionsgut wieder zugeführt werden. Gleichzeitig damit findet ein intensives Waschen der gesamten Reaktorinnenwand mit Inhibitor enthaltender Flüssigphase statt.

Das Versprühen von Anteilen des Reaktionsgutes ist besonders einfach in der Weise möglich, daß absatzweise oder bevorzugt kontinuierlich beschränkte Mengen des flüssigen Reaktionsgutes aus dem Reaktorsumpf abgezogen und über eine Sprühdüse dem mit Gasphase erfüllten Reaktionsinnenraum wieder zugeführt werden. In der technologischen Ausgestaltung bieten sich hier vielfältige Modelle an. So kann mit einer oder mit mehreren Sprühdüsen gearbeitet werden. Der Flüssigkeitskreislauf kann im Reaktorinneren oder derart vorgesehen sein, daß die zu versprühenden Flüssigphasenanteile über eine außerhalb des Reaktors gelegene Pumpe abgezogen und durch die Sprühdüsen dem Reaktorinneren wieder zugeführt werden. Durch die Beschaffenheit der Sprühdüsen und die Art ihres Betriebes kann weitgehend Einfluß auf die Tröpfchengröße und den Vernebelungszustand der Flüssigphase im gaserfüllten Reaktorinneren Einfluß genommen werden.

Wird mit einer solchen Kreislaufführung der Flüssigphase gearbeitet, so kann es zweckmäßig sein, wenigstens einen Anteil des freien Sauerstoff enthaltenden Gasstromes in den abgezogenen Flüssigkeitsteilstrom einzudosieren und zusammen mit diesem wieder dem Reaktorinneren zuzuführen. Es hat sich gezeigt, daß auf diese Weise unerwünschte Blockaden der im Kreislauf geführten Flussigphase vermieden werden können. In einer weiteren wichtigen Ausführungsform wird die Feinverteilung der Flüssigphase im Reaktorinneren derart durchgeführt, daß mit beispielsweise rotierenden, in die flüssige Reaktionsmischung eintauchenden Arbeitselementen Anteile der Flüssigphase im Reaktorinneren hochgesogen und feinverteilt in den mit Gasphase auch eine Mehrzahl solcher rotierenden Arbeitsmittel vorgesehen werden.

In einer besonders wichtigen Ausführungsform der Erfindung wird die Gasphase aus dem Reaktorinneren wenigstens anteilsweise abgesogen und derart wieder in das flüssige Reaktionsgemisch zurückgeführt, daß hierdurch vorbestimmbare Anteile der Flüssigphase vom rückgeführten Gasstrom mitgerissen und in den gaserfüllten Innenraum des Reaktors getragen werden. Wird bei einem solchen Arbeiten die im Kreislauf geführte Gasphase zusätzlich noch getrocknet - d. h. von ihrem Anteil an aufgenommenen Kondensationswasser befreit - so kann hierdurch eine nochmalige Intensivierung und Beschleunigung der Veresterungsreaktion erzielt werden.

In einer besonders bevorzugten Ausführungsform sieht die Erfindung vor, mit bei Reaktionstemperatur flüssigen Reaktionsgemischen zu arbeiten, die wenigstens weitgehend frei von Lösungs- und/oder azeotropen Schleppmitteln sind. Der wirkungsvolle Austrag des bei der Veresterung gebildeten Reaktionswassers gelingt bei der erfindungsgemäßen Verfahrensführung schon durch den Gasstrom, der dem Reaktionsraum zugeführt wird. Es kann dabei jeweils in Abhängigkeit von den Verfahrensbedingungen mit Luft als Gasstrom oder mit Sauerstoff verarmten Gasgemischen - beispielsweise mit Stickstoff/Luft-Gemischen - gearbeitet werden. In aller Regel wird allerdings ein gewisser Gehalt an freiem Sauerstoff in dieser der Reaktionsmischung zugeführten Gasphase gewünscht. Diese beschränkten Sauerstoffmengen aktivieren in an sich bekannter Weise den Inhibitor während des Reaktionsgeschehens.

Durch die erfindungsgemäße Maßnahme, den Polymerisationsinhibitor dem gesamten Reaktorinnenraum über die Teilversprühung des flüssigen, den Inhibitor enthaltenden Reaktionsgutes sicherzustellen, wird es insbesondere auch möglich, den in der praktischen Anwendung der Reaktionsprodukte erwünschten Applikationsinhibitor bereits während der Veresterungsreaktion als Reaktionsinhibitor zu verwenden. Die bis heute übliche Mitverwendung vergleichsweise leicht flüchtiger Inhibitorkomponenten zum hinreichenden Schutz des gas- bzw. dampferfüllten Raumes und der entsprechenden Wandflächen ist nicht mehr erforderlich. Die Erfindung erschließt damit die Möglichkeit, schwer flüchtige aber hoch wirksame Inhibitorsysteme oder entsprechend ausgewählte schwer flüchtige individuelle Inhibitorsubstanzen wirkungsvoll vom Reaktionsbeginn bis zum in der Praxis schließlich erfolgenden Einsatz des Reaktionsproduktes zu verwenden. Ein Austausch des bei der Herstellung eingesetzten Reaktionsinhibitors gegen den in der Anwendung geforderten Applikationsinhibitor ist nicht mehr erforderlich.

Als Polymerisationsinhibitoren können sowohl bestimmt ausgewählte einzelne Inhibitorverbindungen als auch mehrere Komponenten enthaltende Inhibitorsysteme eingesetzt werden. Bevorzugt sind vergleichsweise schwer flüchtige Verbindungen, insbesondere auf Basis einwertiger oder mehrwertiaer Phenole, wobei als mehrwertige Phenoiverbindungen insbesondere zweiwertige Phenoltypen von der Art des Hydrochinons bzw. der Hydrochinonderivate in Betracht kommen.

Für die Herstellung von strahlenhärtbaren (Meth)acrylsäureestern der geschilderten Art mit hoher Reinheit und insbesondere geringer Eigenfarbe kommt im Rahmen der Erfindung besonders drei ausgewählten Typen erhöhte Bedeutung zu. Hierbei handelt es sich zunächst um das Hydrochinon selber, das allerdings bei der Veresterungsreaktion besonderer im nachfolgenden geschilderter Einsatzbedingungen bedarf. Problemloser ist die Anwendung eines sterisch gehinderten Hydrochinons, nämlich des Di-tert.-Butylhydrochinons. Als dritte wichtige Klasse sind zu nennen die sterisch gehinderten Phenolverbindungen von der Art der Tocopherole, wobei hier insbesondere dem alpha-Tocopherol erhebliche Bedeutung zukommt.

Der Einsatz von Di-tert.-butylhydrochinon führt problemlos zu hellfarbigen lagerstabilen (Meth)acrylsäureestern der angestrebten Art. Auch die Verwendung von Tocopherolen insbesondere solchen Gemischen, die wenigstens anteilsweise alpha-Tocopherol enthalten, führt vergleichsweise problemlos zu hellfarbigen Poly-(Meth)acrylsäureestern der gewünschten Art. Treten hier bei der Herstellung insbsondere in Abwesenheit von Lösungsmitteln unter drastischen Veresterungsbedingungen leichte Farbverschlechterungen im Reaktionsprodukt auf, so lassen sich diese problemlos durch eine Nachbehandlung beispielsweise mit Aluminiumoxid beseitigen.

Vergleichsweise größere Farbprobleme fallen bei der lösungsmittelfreien Veresterungsreaktion im Sinne des erfindungsgemäßen Handelns an, wenn das Hydrochinon als Polymerisationsinhibitor eingesetzt wird. Hier treten ohne weitere Hilfsmaßnahmen doch verhältnismäßig starke Verfärbungen im Veresterungsprodukt auf, die durch eine nachträgiiche Behandlung des Reaktionsproduktes nicht ohne weiteres zu entfernen sind. Die Erfindung schlägt in einer besonderen Ausgestaltung vor, diese Problematik dadurch zu beseitigen, daß beim Einsatz von Hydrochinon als Inhibitorkomponente gleichzeitig Aktivkohle dem Reaktionsgemisch zugesetzt wird. Es hat sich überraschenderweise gezeigt, daß ein solcher gemeinsamer Einsatz von Hydrochinon und Aktivkohle unerwünscht starke Verfärbungen des Reaktionsproduktes auch unter drastischen Veresterungsbedingungen unterbindet, andererseits wird durch die Gegenwart der Aktivkohle die Inhibitorwirkung des Hydrochinons nicht substantiell gemindert. Nach Abschluß der Veresterungsreaktion bedarf es dann lediglich der Klärung der Reaktionsmasse beispielsweise durch eine Filtration, um zum weitgehend entfärbten Reaktionsprodukt zu kommen. Die Inhibitoren werden dem Reaktionsgemisch üblicherweise in Mengen von 200 bis 10000 ppm und bevorzugt im Bereich von etwa 300 bis 2000 ppm zugesetzt. Die Zahlenangaben beziehen sich dabei jeweils auf das Gewicht des aus (Meth)acrylsäure und polyfunktionellen Alkoholen bestehenden Reaktionsgemisches.

Wird Hydrochinon in Kombination mit Aktivkohle als Inhibitorsystem eingesetzt so überwiegt in der bevorzugten Ausführungsform die Aktivkohlemenge die Menge an Hydrochinon nicht unbeträchtlich. Zweckmäßigerweise wird die Aktivkohle wenigstens etwa in der zehnfachen Gewichtsmenge eingesetzt wie das Hydrochinon, wobei es bevorzugt sein kann, die Menge der A-Kohle im Bereich des 10- bis 100fachen, bevorzugt im Bereich von etwa dem 20- bis 60fachen der Hydrochinonmenge zu wählen.

Einzelheiten zum Arbeiten mit den hier als bevorzugt aufgezählten Inhibitoren finden sich in den parallelen Patentanmeldungen ... (D 8492, D 8493, D 8494 "Verfahren zur verbesserten Herstellung von (Meth)acrylsäureestern mehrwertiger Alkohole (I - III)"), deren Inhalt hiermit gleichzeitig zum Gegenstand der vorliegenden Erfindungsoffenbarung gemacht wird.

Als zu veresternde Polyalkohole seien beispielsweise genannt: Ethylenglycol, Propylenglycol, Butandiol-1,4, Hexandiol-1,6, Neopentylglycol, Diethylenglycol, Triethylenglycol, Dimethylolpropan, Glycerin, Trimethylolpropan, Trimethylolhexan, Trimethylolethan, Hexantriol-1,3,5 und Pentraerytrit. Erfindungsgemäß kommen als polyfunktionelle Alkohole insbesondere aber auch die Oxalkylierungsprodukte dieser zuvor genannten polyfunktionellen Alkohole in Betracht, wobei hier den Oxethylierungsprodukten und/oder den Oxpropylierungsprodukten besondere Bedeutung zukommt. Kettenverlängerte polyfunktionelle Alkohole dieser Art können beträchtliche Mengen an Polyalkoxidresten enthalten, beispielsweise 1 bis 50 Mol, vorzugsweise etwa 1 bis 20 Mol Ethylenoxid pro g-Äquivalent Hydroxylgruppen.

Veresterungskatalysatoren für das erfindungsgemaße Herstellungsverfahren sind handelsübliche organische oder anorganische Säuren oder auch saure Ionenaustauscher, wobei den in der Praxis häufig eingesetzten entsprechenden Verbindungen p-Toluolsulfonsäure und Schwefelsäure besondere Bedeutung zukommt. Die Mengen des Veresterungskatalysators liegen beispielsweise im Bereich von 0,1 bis 5 Gew.-% bezogen auf das Veresterungsgemisch.

Bei der Umsetzung der Reaktanten kann in Abwesenheit von Lösungs- bzw. Verdünnungsmitteln unter vergleichsweise drastischen Bedingungen gearbeitet werden. Bevorzugt sind hier für die Veresterung Sumpftemperaturen von wenigstens etwa 90 ^{o}C und vorzugsweise von wenigstens etwa 100 ^{o}C, wobei insbesondere der Temperaturbereich bis etwa 150 ^{o}C geeignet ist. Dabei kann unter Normaldruck zweckmäßigerweise aber auch unter abgesenktem Druck gearbeitet werden. Beim Arbeiten mit vermindertem Druck kann in einer besonderen Ausführungsform der Druck in Richtung auf niedrigere Drucke stufenweise oder kontinuierlich variiert werden.

Durch die Möglichkeit unter vergleichsweise scharfen Veresterungsbedingungen und gleichzeitig vermindertem Druck zu arbeiten, wird die Reaktionsdauer gegenüber bisher beschriebenen Verfahren stark abgekürzt. So können im erfindungsgemäßen Verfahren Umsatzausbeuten von wenigstens 90 % der Theorie und vorzugsweise von wenigstens etwa 94 % der Theorie im Temperaturbereich von etwa 100 bis 140 ^{o}C bei einer Reaktionsdauer von nicht mehr als etwa 10 Stunden und vorzugsweise von nicht mehr als etwa 8 Stunden erzielt werden. Gleichwohl fallen die Reaktionsprodukte als hellfarbige oder durch eine einfache Nachbehandlung wirkungsvoll zu reinigende stabilisierte Masse an.

Das den sauren Veresterungskatalysator enthaltende Reaktionsrohprodukt wird einer nachfolgenden Neutralisation unterworfen. Diese Neutralisation kann unter bekannten Naßbedingungen, beispielsweise durch Einsatz von wäßrigen Soda- und gegebenenfalls Natriumchlorid enthaltenden Lösungen erfolgen. In einer bevorzugten Ausführungsform wird allerdings das den sauren Katalysator enthaltende Reaktionsrohprodukt einer trockenen Neutralisation unterworfen. Geeignete trockene Neutralisationsmittel sind die Oxide und/oder Hydroxide der Alkalimetalle, der Erdalkalimetalle, insbesondere des Magnesiums bzw. Calciums und/oder des Aluminiums.

(Meth)acrylsäure und die Alkohole können für die Veresterung in äquivalenten Mengenverhältnissen eingesetzt werden. Bei den mehr als zweiwertigen Alkoholen ist es allerdings auch ohne weiteres möglich, nur einen Teil der Hydroxylgruppen zu verestern. Zur Vollveresterung kann es zweckmäßig sein, die Säurekomponente in leichtem Überschuß über die zur Veresterung der Hydroxylgruppen erforderliche stöchiometrische Menge einzusetzen. Ein solcher Überschuß kann wenigstens etwa 10 Mol-% ausmachen. Nach Abschluß der Reaktion kann gewünschtenfalls zusätzlich ein Inhibitor dem Reaktionsprodukt beigemischt werden.

### Beispiele

### Beispiel 1

In einem 30-Liter-Reaktor wurden 14,53 kg Acrylsäure, 14,18 kg eines ethoxylierten Trimethylolpropans (OH-Zahl: 665 mg KOH/g Substanz), 1,01kg p-Toluolsulfonsäure sowie 0,047 kg 2 ,5-Di-tert. -butylhydrochinon (2000 ppm bezogen auf Produktmenge) eingewogen. Die Veresterung wurde unter Durchleiten von Luft (100 l/h) und unter Wasserabtrennung durchgeführt. Das inhibierte Reaktionsgemisch wurde mittels einer Druckluftmembranpumpe und einer Einstoffdüse im oberen, nichtinhibierten Reaktorraum versprüht. Der Produktstrom (40 l/h) wurde dabei so gewählt, daß alle Reaktorteie mit inhibiertem Produkt benetzt waren. Zur Vermeidung unerwünschter Polymerisatbildung erfolgte die Teflonbeschichtung der Innenwände der Rohrleitungen und die zusätzliche Lufteinspeisung (60 l/h) in den Druckteil des Produktkreislaufes. Als weitere Maßnahme zur Inhibierung des Kondensationsteiles des Reaktors wurde das zusätzlich inhibierte Destillat im Kreislauf geführt und in dem Kondensatorbereich versprüht. Bei einer maximalen Reaktionstemperatur von 105 ^{o}C und einem Vakuumprofil von 2 h/400 mbar; 1 h/300 mbar; 0,5 h/200 mbar; 1 h/100 mbar und 0,5 h/23 mbar betrug die Veresterungszeit 5 Stunden.

| Rohprodukt: | |
|---|---|
| Säurezahl: | 17,3 mg KOH/g |
| OH-Zahl: | 12,6 mg KOH/g |
| Umsatz: | 96,9 % |
| Gardner Farbzahl: | < 1 |
| Viskosität: | 150 mPas |
| H₂O-Gehalt: | 0,09 % |

Das Rohprodukt wurde durch Zugabe von 0,53 kg Ca(OH)₂ und 2stündigem Rühren bei 80 ^{o}C und 50 mbar neutralisiert und anschließend mit einer Druckfilternutsche filtriert.

| Produkt: | |
|---|---|
| Säurezahl: | < 1 mg KOH/g |
| OH-Zahl: | 14 mg KOH/g |
| Gardner Farbzahl: | < 1 |

### Vergleichsbeispiel 1

Beispiel 1 wurde wiederholt mit der Änderung, daß während der Veresterung auf das Versprühen von Produkt und Destillat verzichtet wurde. Dabei wurde eine deutliche Polymerisatbildung am Reaktordeckel und im Kondensationsbereich schon nach 30-minütiger Veresterungszeit beobachtet.

| Rohprodukt: | |
|---|---|
| Säurezahl: | 39,2 mg KOH/g |
| OH-Zahl: | 18 mg KOH/g |
| Umsatz: | 95,4 % |
| Gardner Farbzahl: | 1 |
| H₂O-Gehalt: | 0,13 % |

Das Rohprodukt wurde durch Zugabe von 1,5 kg Ca(OH)₂ und 2-stündigem Rühren bei 80 ^{o}C und 50 mbar neutralisiert und anschließend mit einer Druckfilternutsche filtriert.

| Produkt: | |
|---|---|
| Säurezahl: | < 1 mg KOH/g |
| OH-Zahl: | 21 mg KOH/g |
| Gardner Farbzahl: | < 1 |
| H₂O-Gehalt: | 0,17 % |

### Bespiel 2

12,97 kg Acrylsäure, 15,82 kg eines propoxylierten Neopentylglycols (OH-Zahl: 509 mg KOH/g Substanz) sowie 1,01kg p-Toluolsulfonsäure wurden in einen 30-Liter-Reaktor eingewogen und mit 47 g 2,5-Di-tert.-butylhydrochinon (2000 ppm bezogen auf Produktmenge) inhibiert. Unter Durchleiten von Luft (100 l/h) wurde die Veresterung unter Wasserabtrennung durchgeführt. Mittels einer Druckluftmembranpumpe und einer Einstoffdüse wurde ein Teil des inhibierten Reaktionsgemisches im oberen, nicht-inhibierten Reaktorraum versprüht. Bei einem Produktstrom von 40 l/h waren alle Reaktorteile mit inhibiertem Produkt benetzt. Zur Vermeidung unerwünschter Polymerisatbildung erfolgte die Teflonbeschichtung der Innenwände der Rohrleitungen und eine Lufteinspeisung (60 l/h) in den Druckte des Produktkreislaufes. Als weitere Marßnahme zur Inhibierung des Kondensationsteiles des Reaktors wurde das zusätzlich inhibierte Destillst im Kreislauf geführt und in dem Kondensatorbereich versprüht. Bei einer maximalen Reaktionstemperatur von 105 ^{o}C und einem Vakuumprofil von 2 h/400 mbar; 1 h/300 mbar; 0,5 h/200 mbar; 1 h/100 mbar und 0,5 h/30 mbar betrug die Veresterungszeit 5 Stunden.

| Rohprodukt: | |
|---|---|
| Säurezahl: | 34 mg KOH/g |
| OH-Zahl: | 17 mg KOH/g |
| Umsatz: | 94,4 % |
| Gardner Farbzahl: | < 1 |

Das Rohprodukt wurde durch Zugabe von 1,3 kg Ca(OH)₂ und 2-stündigem Rühren bei 80^{o}C und 50 mbar neutralisiert und anschließend mit einer Druckfilternutsche filtriert.

| Produkt: | |
|---|---|
| Säurezahl: | < 1 mg KOH/g |
| OH-Zahl: | 20 mg KOH/g |
| Gardner Farbzahl: | < 1 |

### Vergleichsbeispiel 2

Beispiel 2 wurde wiederholt mit der Änderung, daß während der Veresterung auf das Versprühen von Produkt und Destillat verzichtet wurde. Dabei trat eine deutliche Polymerisatbildung am Reaktordeckel und im Kondensationsbereich schon nach 40-minütiger Veresterungszeit auf.

| Rohprodukt: | |
|---|---|
| Säurezahl: | 20 mg KOH/g |
| OH-Zahl: | 20 mg KOH/g |
| Umsatz: | 95,0 % |
| Gardner Farbzahl: | 1 |

Das Rohprodukt wurde durch Zugabe von 0,63 kg Ca(OH)₂ und 1,5-stündigem Rühren bei 80 ^{o}C und 50 mbar neutralisiert und anschiießend mit Hilfe einer Druckfilternutsche filtriert.

| Produkt: | |
|---|---|
| Säurezahl: | < 1 mg KOH/g |
| OH-Zahl: | 23 mg KOH/g |
| Gardner Farbzahl: | < 1 |

### Beispiel 3

14,53 kg Acrylsäure, 14,18 kg eines ethoxylierten Trimethylolpropans (OH-Zahl: 665 mg KOH/g Substanz), 1,01 kg p-Toluolsulfonsäure wurden in einen 30-Liter-Reaktor eingewogen und mit 52,6 g Alpha-Tocopherol (Fa. Henkel; 2000 ppm bezogen auf Produktmenge) inhibiert. Unter Durchleiten von Luft (100 l/h) wurde die Veresterung unter Wasserabtrennung durchgeführt. Mittels einer Druckluftmembranpumpe und einer Einstoffdüse wurde ein Teil des inhibierten Reaktionsgemisches im oberen, nicht-inhibierten Reaktorraum versprüht. Der Produktstrom (40 l/h) wurde dabei so gewählt, daß alle Reaktorteile mit inhibiertem Produkt benetzt waren. Zur Vermeidung unerwünschter Polymerisatbildung erfolgte die Teflonbeschichtung der Innenwände der Rohrleitungen und eine Lufteinspeisung (60 l/h) in den Druckteil des Produktkreislaufes. Als weitere Maßnahme zur Inhibierung des Kondensationsteiles des Reaktors wurde das zusätzlich inhibierte Destillat im Kreislauf geführt und in dem Kondensatorbereich versprüht. Bei einer maximalen Reaktionstemperatur von 105 ^{o}C und einem Vakuumprofil von 2 h/400 mbar; 1 h/300 mbar; 0,5 h/200 mbar; 1 h/100 mbar und 0,5 h/25 mbar betrug die Veresterungszeit 5 Stunden.

| Rohprodukt: | |
|---|---|
| Säurezahl: | 17,0 mg KOH/g |
| OH-Zahl: | 33,6 mg KOH/g |
| Umsatz: | 91,4 % |
| Gardner Farbzahl: | 7 - 8 |
| H₂O-Gehalt: | 0,16 % |

Das Rohprodukt wurde durch Zugabe von 0,52 kg Ca(OH)₂ und 1 ,5-stündigem Rühren bei 80 ^{o}C und 50 mbar neutralisiert und anschließend mit Hilfe einer Druckfilternutsche filtriert.

| Produkt: | |
|---|---|
| Säurezahl: | < 1 mg KOH/g |
| OH-Zahl: | 43 mg KOH/g |
| Gardner Farbzahl: | 5 - 6 |
| H₂O-Gehalt: | 0,42 % |

Zur Entfärbung wurde das neutralisierte und filtrierte Produkt mit 2,5 kg basischem Al₂O₃ 2 Stunden bei 80 ^{o}C und 50 mbar gerührt und anschließend mit Hilfe einer Druckfilternutsche filtriert.

| Produkt: | |
|---|---|
| Säurezahl: | < 1 mg KOH/g |
| OH-Zahl: | 42 mg KOH/g |
| Gardner Farbzahl: | < 1 |
| H₂O-Gehalt: | 0,08 % |

### Vergleichsbeispiel 3

Beispiel 3 wurde wiederholt mit der Änderung, daß während der Veresterung auf das Versprühen von Produkt und Destillat verzichtet wurde. Es kam bereits nach 30-minütiger Veresterungszeit zur deutlichen Polymerisatbildung am Reaktordeckel und im Kondensationsbereich.

| Rohprodukt: | |
|---|---|
| Säurezahl: | 23 mg KOH/g |
| OH-Zahl: | 19 mg KOH/g |
| Umsatz: | 95,3 % |
| Gardner Farbzahl: | 8 |

Das Rohprodukt wurde durch Zugabe von 0,71 kg Ca(OH)₂ und 1-stündigem Rühren bei 80 ^{o}C und 50 mbar neutralisiert und anschließend mit Hilfe einer Druckfilternutsche filtriert.

| Neutralisiertes Produkt: | |
|---|---|
| Säurezahl: | < 1 mg KOH/g |
| OH-Zahl: | 20 mg KOH/g |
| Gardner Farbzahl: | 6 |

Das neutralisierte und filtrierte Produkt wurde zur Entfärbung 2 Stunden bei 80 ^{o}C mit 2,5 kg basischem Al₂O₃ gerührt und anschließend mit Hilfe einer Druckfilternutsche filtriert.

| Produkt: | |
|---|---|
| Säurezahl: | < 1 mg KOH/g |
| OH-Zahl: | 22 mg KOH/g |
| Gardner Farbzahl: | < 1 |

### Beispiel 4

12,97 kg Acrylsäure, 15,82 kg eines propoxylierten Neopentylglycols (OH-Zahl: 509 mg KOH/g Substanz) sowie 1,01 kg p-Toluolsulfonsäure wurden in einen 30-Liter-Reaktor eingewogen und mit 47 g Alpha-Tocopherol (Fa. Henkel; 2000 ppm bezogen auf Produktmenge) inhibiert. Unter Durchleiten von Luft (40 l/h) wurde die Veresterung unter Wasserabtrennung durchgeführt. Mittels einer Druckluftmembranpumpe wurde ein Teil des inhibierten Reaktionsgemisches geführt und über eine Einstoffdüse im oberen nicht-inhibierten Reaktorraum versprüht. Bei einem Produktstrom von 40 l/h waren alle Reaktorteile mit inhibiertem Produkt benetzt. Zur Vermeidung unerwünschter Polymerisatbildung erfolgte die Teflonbeschichtung der Innenwände der Rohrleitungen und eine Lufteinspeisung (60 l/h) in den Druckteil des Produktkreislaufes. Als weitere Maßnahme zur Inhibierung des Kondensationsteiles des Reaktors wurde ein Teil des zusätzlich inhibierten Destillats im Kreislauf geführt und in dem Kondensatorbereich versprüht. Bei einer maximalen Reaktionstemperatur von 105 ^{o}C und einem Vakuumprofil von 2/400 mbar; 1 h/300 mbar; 0,5 h/200 mbar; 1 h/100 mbar und 0,5 h/ 25 mbar betrug die Veresterungszeit 5 Stunden.

| Rohprodukt: | |
|---|---|
| Säurezahl: | 24 mg KOH/g |
| OH-Zahl: | 20 mg KOH/g |
| Umsatz: | 94,4 % |
| Gardner Farbzahl: | 7 - 8 |

Das Rohprodukt wurde durch Zugabe von 0,72 kg Ca(OH)₂ und Istündigem Rühren bei 80 ^{o}C und 50 mbar neutralisiert und anschließend mit Hilfe einer Druckfilternutsche filtriert.

| Neutralisiertes Produkt: | |
|---|---|
| Säurezahl: | < 1 mg KOH/g |
| OH-Zahl: | 22 mg KOH/g |
| Gardner Farbzahl: | 6 |

Zur Entfärbung wurde das neutralisierte und filtrierte Produkt mit 2,5 kg basischem Al₂O₃ 2 Stunden bei 80 ^{o}C gerührt und anschließend mit Hilfe einer Druckfilternutsche filtriert.

| Produkt: | |
|---|---|
| Säurezahl: | < 1 mg KOH/g |
| OH-Zahl: | 24 mg KOH/g |
| Gardner Farbzahl: | < 1 |

### Vergleichsbeispiel 4

Beispiel 4 wurde wiederholt mit der Änderung, daß während der Veresterung auf das Versprühen von Produkt und Destillat verzichtet wurde. Es kam bereits nach 30 Minuten Veresterungszeit zur deutlichen Polymerisatbildung am Reaktordeckel und im Kondensationsbereich.

| Rohprodukt: | |
|---|---|
| Säurezahl: | 35,1 mg KOH/g |
| OH-Zahl: | 25,2 mg KOH/g |
| Umsatz: | 92,6 % |
| Gardner Farbzahl: | 7 - 8 |

Das Rohprodukt wurde durch Zugabe von 1,09 kg Ca(OH)₂ und 1-stündigem Rühren bei 80 ^{o}C und 50 mbar neutralisiert und anschließend mit Hilfe einer Druckfilternutsche filtriert.

| Neutralisiertes Produkt: | |
|---|---|
| Säurezahl: | < 1 mg KOH/g |
| OH-Zahl: | 28 mg KOH/g |
| Gardner Farbzahl: | 5 |

Zur Entfärbung wurde das neutralisierte und filtrierte Produkt mit 2,5 kg basischem Al₂O₃ 2 Stunden bei 80 ^{o}C gerührt und anschließend mit Hilfe einer Druckfilternutsche filtriert.

| Produkt: | |
|---|---|
| Säurezahl: | < 1 mg KOH/g |
| OH-Zahl: | 28 mg KOH/g |
| Gardner Farbzahl: | < 1 |

### Beispiel 5

1559,5 g Acrylsäure, 1521,0 g eines ethoxylierten Trimethylolpropans (OH-Zahl 665 mg KOH/g Substanz) sowie 107,8 g p-Toluolsulfonsäure und 124,4 g (5 Gew.-% bezogen auf Acrylsäure + Polyol) A-Kohle wurden in einen mit einem Rührer versehenen 3-Liter-Reaktor eingewogen und mit 2,5 g Hydrochinon (1100 ppm bezogen auf Produktmenge) inhibiert. Unter Durchleiten von Luft (40 l/h) wurde die Veresterung unter Wasserabtrennung durchgeführt. Bei einer Rührerdrehzahl von 500 U/min wurde der gesamte Deckelbereich des Reaktors mit inhibierter Produktmischung benetzt, so daß es zu keinerlei unerwünschter Polymerisatbildung kam. Bei einer maximalen Reaktionstemperatur von 105 ^{o}C und einem Vakuumprofil von 2 h/400 mbar; 1 h/300 mbar; 1 h/150 mbar; 1 h/40 mbar betrug die Veresterungszeit 5 Stunden. Der Ansatz wurde auf 80 ^{o}C abgekühlt und mit Hilfe einer Druckfilternutsche filtriert.

| Rohprodukt: | |
|---|---|
| Säurezahl: | 24,8 mg KOH/g |
| OH-Zahl: | 23,8 mg KOH/g |
| Umsatz: | 94,1 % |
| Gardner Farbzahl: | < 1 |

Das Rohprodukt wurde durch Zugabe von 82 g Ca(OH)₂ und 1-stündigem Rühren bei 80 ^{o}C und 50 mbar neutralisiert und anschließend mit Hilfe einer Druckfilternutsche filtriert.

| Produkt: | |
|---|---|
| Säurezahl: | < 1 mg KOH/g |
| OH-Zahl: | 29 mg KOH/g |
| Gardner Farbzahl: | < 1 |

### Vergleichsbeispiel 5

Beispiel 5 wurde wiederholt mit der Änderung, daß die Rührerdrehzahl 150 U/min betrug. Da es zu keiner Benetzung des Reaktordeckels mit inhibiertem Produkt kam, wurde eine deutliche Polymerisatbildung an den unbenetzten Reaktorteilen beobachtet.

| Rohprodukt: | |
|---|---|
| Säurezahl: | 28 mg KOH/g |
| OH-Zahl: | 17 mg KOH/g |
| Umsatz: | 95,8 % |
| Gardner Farbzahl: | 1 |

Das Rohprodukt wurde durch Zugabe von 92 g Ca(OH)₂ und 1-stündigem Rühren bei 80 ^{o}C und 50 mbar neutralisiert und anschließend mit Hilfe einer Druckfilternutsche filtriert.

| Produkt: | |
|---|---|
| Säurezahl: | < 1 mg KOH/g |
| OH-Zahl: | 23 mg KOH/g |
| Gardner Farbzahl: | 1 |

### Beispiel 6

1559,5 g Acrylsäure, 1521,0 g eines ethoxylierten Trimethylolpropans (OH-Zahl 665 mg KOH/g Substanz) sowie 107,8 g p-Toluolsulfonsäure wurden in einen 3-Liter-Reaktor eingewogen und mit 4,96 g 2,5-Di-tert.-butylhydrochinon (2000 ppm bezogen auf Produktmenge) inhibiert. Unter Durchleiten von Luft (40 l/h) wurde die Veresterung unter Nasserabtrennung durchgeführt. Bei einer Rührerdrehzahl von 500 U/min wurde der gesamte Deckelbereich des Reaktors mit inhibierter Produktmischung benetzt, so daß es zu keinerlei unerwünschter Polymerisatbildung kam. Bei einer maximalen Reaktionstemperatur von 105 ^{o}C und einem Vakuumsprofil von 2 h/400 mbar; 1 h/300 mbar; 1 h/150 mbar; 1 h/40 mbar betrug die Veresterungszeit 5 Stunden.

| Rohprodukt: | |
|---|---|
| Säurezahl: | 32 mg KOH/g |
| OH-Zahl: | 21 mg KOH/g |
| Umsatz: | 94,8 % |
| Gardner Farbzahl: | < 1 |

Das Rohprodukt wurde durch Zugabe von 105 g Ca(OH)₂ und 1-stündigem Rühren bei 80 ^{o}C und 50 mbar neutralisiert und anschließend mit Hilfe einer Druckfilternutsche filtriert.

| Produkt: | |
|---|---|
| Säurezahl: | < 1 mg KOH/g |
| OH-Zahl: | 25 mg KOH/g |
| Gardner Farbzahl: | < 1 |

### Vergleichsbeispiel 6

Beispiel 6 wurde wiederholt mit der Änderung, daß es bei einer Rührerdrehzahl 150 U/min zu keiner Benetzung des Reaktordeckels mit inhibiertem Produkt kam. Es wurde eine deutliche Polymerisatbildung an den unbenetzten Reaktorteilen beobachtet.

| Rohprodukt: | |
|---|---|
| Säurezahl: | 15 mg KOH/g |
| OH-Zahl: | 14 mg KOH/g |
| Umsatz: | 96,5 % |
| Gardner Farbzahl: | < 1 |

Das Rohprodukt wurde durch Zugabe von 46 g Ca(OH)₂ und 1-stündigem Rühren bei 80 ^{o}C und 50 mbar neutralisiert und anschließend mit Hilfe einer Druckfilternutsche filtriert.

| Produkt: | |
|---|---|
| Säurezahl: | < 1 mg KOH/g |
| OH-Zahl: | 18 mg KOH/g |
| Gardner Farbzahl: | < 1 |

### Beispiel 7

1297 g Acrylsäure, 1582 g eines propoxylierten Neopentylglycols (OH-Zahl: 509 mg KOH/g Substanz) sowie 101 g p-Toluolsulfonsäure wurden in einen 3-Liter-Reaktor eingewogen und mit 4,79 g Alpha-Tocopherol (Fa. Henkel) inhibiert. Unter Durchleiten von Luft (40 l/h) wurde die Veresterung unter Wasserabtrennung durchgeführt. Bei einer Rührerdrehzahl von 500 U/min wurde der gesamte Deckelbereich des Reaktors mit inhibierter Produktmischung benetzt, so daß es zu keinerlei unerwünschter Polymerisatbildung kam. Bei einer maximalen Reaktionstemperatur von 105 ^{o}C und einem Vakuumsprofil von 2 h/400 mbar; 1 h/300 mbar; 1 h/150 mbar; 1 h/40 mbar betrug die Veresterungszeit 5 Stunden.

| Rohprodukt: | |
|---|---|
| Säurezahl: | 15 mg KOH/g |
| OH-Zahl: | 10 mg KOH/g |
| Umsatz: | 97,0 % |
| Gardner Farbzahl: | 7 - 8 |

Das Rohprodukt wurde durch Zugabe von 47 g Ca(OH)₂ und 1-stündigem Rühren bei 80 ^{o}C und 50 mbar neutralisiert und anschließend mit Hilfe einer Druckfilternutsche filtriert.

| Neutralisiertes Produkt: | |
|---|---|
| Säurezahl: | < 1 mg KOH/g |
| OH-Zahl: | 12 mg KOH/g |
| Gardner Farbzahl: | 6 |

Das neutralisierte und filtrierte Produkt wurde zur Entfärbung mit 240 g basischem Al₂O₃ bei 80 ^{o}C gerührt und anschließend mit einer Druckfilternutsche filtriert.

| Produkt: | |
|---|---|
| Säurezahl: | < 1 mg KOH/g |
| OH-Zahl: | 12 mg KOH/g |
| Gardner Farbzahl: | 1 |

### Vergleichsbeispiel 7

Beispiel 7 wurde wiederholt mit der Änderung, daß es bei einer Rührerdrehzahl von 150 U/min zu keiner Benetzung des Reaktordeckels mit inhibiertem Produkt kam. Es wurde eine deutliche Polymerisatbildung an den unbenetzten Reaktorteilen beobachtet.

| Rohprodukt: | |
|---|---|
| Säurezahl: | 25 mg KOH/g |
| OH-Zahl: | 25 mg KOH/g |
| Umsatz: | 92,6 % |
| Gardner Farbzahl: | 7 - 8 |

Das Rohprodukt wurde durch Zugabe von 79g Ca(OH)₂ und 1,5-stündigem Rühren bei 80 ^{o}C und 50 mbar neutralisiert und anschließend mit Hilfe einer Druckfilternutsche filtriert.

| Neutralisiertes Produkt: | |
|---|---|
| Säurezahl: | < 1 mg KOH/g |
| OH-Zahl: | 30 mg KOH/g |
| Gardner Farbzahl: | 5 |

Das neutralisierte und filtrierte Produkt wurde zur Entfärbung mit 240 g basischem Al₂O₃ bei 80 ^{o}C gerührt und anschließend mit Hilfe einer Druckfilternutsche filtriert.

| Produkt: | |
|---|---|
| Säurezahl: | < 1 mg KOH/g |
| OH-Zahl: | 29 mg KOH/g |
| Gardner Farbzahl: | < 1 |

## Patentansprüche

1. Verfahren zur Herstellung von (Meth)acrylsäureestern mehrwertiger Alkohole durch Umsetzung der Reaktanten in Gegenwart von sauren Veresterungskatalysatoren unter Zusatz von Polymerisationsinhibitoren zum Reaktionsgemisch und Durchspülen des Reaktionsraumes mit einem Sauerstoff enthaltenden Gasstrom, dadurch gekennzeichnet, daß man den mit Gasphase erfüllten Anteil des Reaktionsinnenraumes mit feinverteilten Flüssigkeitströpfchen belädt, die Polymerisationsinhibitor enthalten.

2. Verfahren nach Anspruch 1, dadurch gekennzeichnet, daß man die Inhibitor enthaltende fein-disperse Flüssigphase in solcher Menge dem die Gasphase enthaltenden Reaktionsinnenraum zuführt, daß alle von der Gasphase berührten Festkörperinnenflächen mit einem bevorzugt geschlossenen Inhibitor enthaltenden Flüssigkeitsfilm benetzt sind.

3. Verfahren nach Ansprüchen 1 und 2, dadurch gekennzeichnet, daß man Anteile des flüssigen, den Polymerisationsinhibitor enthaltenden Reaktionsgemiches absatzweise oder kontinuierlich fein-versprüht und damit den mit Gasphase erfüllten Reaktionsinnenraum belädt, die Reaktionsinnenwände mit den daran abfließenden Flüssigkeitsfilmen wäscht und diese Anteile des Reaktorgutes bevorzugt mit der geschlossenen Flüssigphase wieder vereinigt.

4. Verfahren nach Ansprüchen 1 bis 3, dadurch gekennzeichnet, daß man mit bei Reaktionstemperatur flüssigen Reaktionsgemischen arbeitet, die wenigstens weitgehend frei von Lösungs- und/oder azeotropen Schleppmitteln sind.

5. Verfahren nach Ansprüchen 1 bis 4, dadurch gekennzeichnet, daß man den im praktischen Einsatz des Reaktionsproduktes gewünschten Polymerisationsinhibitor (Applikationsinhibitor) bereits während der Veresterungsreaktion als Reaktionsinhibitor verwendet.

6. Verfahren nach Ansprüchen 1 bis 5, dadurch gekennzeichnet, daß man Phenolverbindungen als Polymerisationsinhibitoren einsetzt, wobei sterisch nicht gehinderte Phenole, insbesondere Hydrochinon, bevorzugt zusammen mit Aktivkohle während der Veresterungsreaktion zum Einsatz kommen, während sterisch gehinderte Phenolverbindungen auch ohne Zusatz von Aktivkohle verwendet werden können.

7. Verfahren nach Ansprüchen 1 bis 6, dadurch gekennzeichnet, daß als sterisch gehinderte Phenolverbindungen Tocopherole, bevorzugt wenigstens anteilsweise alpha-Tocopherol und/oder Di-tert.-butylhydrochinon zum Einsatz kommen.

8. Verfahren nach Ansprüchen 1 bis 7, dadurch gekennzeichnet, daß die Veresterung bei Sumpftemperaturen von wenigstens etwa 90 ^{o}C, vorzugsweise von wenigstens etwa 100 ^{o}C und insbesondere im Bereich bis etwa 150 ^{o}C durchgeführt wird, wobei bevorzugt wenigstens absatzweise bei vermindertem, gegebenfalls stufenweise zunehmend vermindertem Druck gearbeitet wird.

9. Verfahren nach Ansprüchen 1 bis 8, dadurch gekennzeichnet, daß der Reaktionsraum mit Luft oder Stickstoff/Luft-Gemischen durchspült wird.

10. Verfahren nach Ansprüchen 1 bis 9, dadurch gekennzeichnet, daß die Inhibitoren in Mengen von 200 bis 10000 ppm, bevorzugt im Bereich von 300 bis 2000 ppm - jeweils bezogen auf das Gewicht des Reaktionsgemisches - eingesetzt werden, wobei das Arbeiten mit unter Reaktionsbedingungen schwer flüchtigen Inhibitoren bevorzugt ist.

11. Verfahren nach Ansprüchen 1 bis 10, dadurch gekennzeichnet, daß die Reaktion auf einen Umsatz von wenigstens 90 % der Theorie, vorzugsweise von wenigstens 94 % der Theorie geführt, wobei bevorzugt im Temperaturbereich von etwa 100 bis 140 ^{o}C, gegebenenfalls unter Vakuum für eine Reaktionsdauer von nicht mehr als 10 Stunden, insbesondere von nicht mehr als 8 Stunden gearbeitet wird.

12. Verfahren nach Ansprüchen 1 bis 11, dadurch gekennzeichnet, daß das Reaktionsrohprodukt einer trockenen Neutralisation - bevorzugt mit Oxiden und/oder Hydroxiden der Erdalkalimetalle und/oder des Aluminiums - unterworfen wird.

13. Verfahren nach Ansprüchen 1 bis 12, dadurch gekennzeichnet, daß die primär anfallenden Reaktionsprodukte einer abschließenden Behandlung mit Entfärbungsmitteln unterworfen werden.

## Claims

1. A process for the production of (meth)acrylic acid esters of polyhydric alcohols by reaction of the reactants in the presence of acidic esterification catalysts with the addition of polymerization inhibitors to the reaction mixture and purging the reaction volume with an oxygen-containing gas stream, characterized in that the part of the reactor interior filled with gas phase is charged with finely divided liquid droplets containing polymerization inhibitor.

2. The process according to claim 1, characterized in that the inhibitor-containing finely disperse liquid phase is introduced in such a quantity into the reactor interior containing the gas phase that all inner solid surfaces contacted by the gas phase are wetted with a preferably continuous inhibitor-containing liquid film.

3. The process according to claims 1 and 2, characterized in that portions of the liquid reaction mixture containing the polymerization inhibitor are finely atomized batchwise or continuously and, thus, charge the interior of the reactor filled with gas phase, the inner walls of the reactor are washed by the liquid films flowing downwardly on said walls, and these portions of the material in the reactor are preferably recombined with the continuous liquid phase.

4. The process according to claims 1 to 3, characterized in that the reaction mixtures are liquid at room temperature and are at least largely free from solvents and/or azeotropic entraining agents.

5. The process according to claims 1 to 4, characterized in that the polymerization inhibitor (application inhibitor) desired in the practical application of the reaction product is used as the reaction inhibitor during the esterification reaction.

6. The process according to claims 1 to 5, characterized in that phenol compounds are used as polymerization inhibitors, with sterically unhindered phenols, particularly hydroquinone, being used preferably together with activated carbon during the esterification reaction, while sterically hindered phenol compounds may also be used with no activated carbon added.

7. The process according to claims 1 to 6, characterized in that tocopherols, preferably at least partly α-tocopherol and/or di-tert.-butylhydroquinone, are used as the sterically hindered phenol compounds.

8. The process according to claims 1 to 7, characterized in that the esterification reaction is carried out at bottoms temperatures of at least about 90 °C, preferably of at least about 100 °C and, more preferably, in the range up to about 150 °C, the reaction preferably being carried out at least batchwise under reduced pressure and optionally under a pressure increasingly reduced in steps.

9. The process according to claims 1 to 8, characterized in that the reaction volume is purged with air or with mixtures of nitrogen and air.

10. The process according to claims 1 to 9, characterized in that the inhibitors are used in quantities of from 200 to 10,000 ppm, and preferably in quantities of from 300 to 2,000 ppm, based on the weight of the reaction mixture, with the use of inhibitors of low volatility under the reaction conditions being preferred.

11. The process according to claims 1 to 10, characterized in that the reaction is continued to a coversion of at least 90% of the theoretical, and preferably of at least 94% of the theoretical, while it is preferably carried out at a temperature in the range from about 100 to 140 °C, optionally *in vacuo*, over a reaction time of not more than 10 hours and, in particular, not more than 8 hours.

12. The process according to claims 1 to 11, characterized in that the crude reaction product is subjected to a dry neutralization, preferably using oxides and/or hydroxides of the alkaline earth metals and/or of aluminum.

13. The process according to claims 1 to 12, characterized in that the primary reaction products obtained are subjected to a final treatment with decolorizing agents.

## Revendications

1. Procédé d'obtention d'esters d'aide (meth) acrylique avec des alcools plurivalents par mise en réction des réactifs en présence de catalyseurs acides d'estérification tout en ajoutant des inhibiteurs de polymérisation au mélange réactionnel et lavage complet de l'espace réactionnel avec un courant gazeux contenant de l'oxygène, caractérisé en ce que l'on charge la fraction remplie de la phase gazeuse avec des gouttelettes finement divisées de liquide qui contiennent un inhibiteur de polymérisation.

2. Procédé selon la revendication 1, caractérisé en ce que l'on amène la phase liquide finement dispersée qui contient l'inhibiteur en quantité telle à l'espace interne de réaction contenant la phase gazeuse, que toutes les surfaces internes des solides mis en contact avec la phase gazeuse sont mouillées avec un film de liquide qui contient un inhibiteur continu.

3. Procédé selon les revendications 1 et 2, caractérisé en ce que l'on pulvérise finement les fractions de mélange réactionnel renfermant l'inhibiteur de polymérisation, graduellement ou en continu et ainsi charge l'espace interne de réaction rempli de phase gazeuse, que l'on lave les parois internes de la réaction avec les films de liquide qui s'en écoulent et que l'on réunit à nouveau ces fractions de produit de réaction de préférence avec la phase liquide continue.

4. Procédé selon les revendications 1 à 3, caractérisé en ce que l'on opère avec des mélanges de réaction liquides à la température de réaction, qui sont au moins largement dépourvus de solvant et/ou d'agent d'entraînement azéotropique.

5. Procédé selon les revendications 1 à 4, caractérisé en ce que l'on utilise l'inhibiteur de polymérisation (inhibiteur d'application) désire dans l'utilisation en pratique du produit réactionnel déjà pendant la réaction d'estérification en tant qu'inhibiteur de réaction._{.}

6. Procédé selon les revendications 1 à 5, caractérisé en ce que l'on met en oeuvre les composés phénoliques comme inhibiteurs de polymérisation, pour lesquels des phénols non empêchés stériquement, en particulier l'hydroquinone de préférence conjointement à du charbon actif viennent en action pendant la réaction d'estérification alors que des composés phénoliques stériquement empêchés peuvent être utilisés également sans addition de charbon actif.

7. Procédé selon les revendications 1 à 6, caractérisé en ce que comme composés phénoliques empêchés stériquement des tocophérols - de préférence au moins par fractions de l' alpha-tocophérol et/ou de la diterbutylhydroquinone viennent en action.

8. Procédé selon les revendications 1 à 7, caractérisé en ce que l'estérification est effectuée à des températures de la cuve d'au moins environ 90°C, de préférence d'au moins environ 100°C et en particulier dans la plage d'environ 150°C pour laquelle de préférence on opère au moins graduellement à pression réduite, le cas échéant à pression progressivement réduite d'une manière croissante.

9. Procédé selon les revendications 1 à 8, caractérisé en ce que l'espace réactionnel est rincé complètement avec de l'air ou des mélanges azote/air.

10. procédé selon les revendications 1 à 9, caractérisé en ce que l'on met en oeuvre les inhibiteurs en quantités allant de 200 à 10.000 ppm - de préférence dans la zone allant de 300 à 2000 ppm à chaque fois rapporté au poids de mélange réactionnel, pour lesquels le travail avec des inhibiteurs difficilement volatils dans les conditions de réaction préféré.

11. Procédé selon les revendications 1 à 10, caractérisé en ce que la réaction est menée à une conversion d'au moins 90 % de la théorie, de préférence d'au moins de 94 % de la théorie, pour laquelle on opère de préférence dans une plage de températures environ de 100 à 140°C le cas échéant sous vide pour une durée de réaction de pas plus de 10 heures, en particulier de pas plus de 8 heures.

12. Procédé selon les revendications 1 à 11, caractérisé en ce que le produit de la réaction est soumis à une neutralisation à sec - de préférence avec des oxydes et/ou des hydroxydes de métal alcalino-terreux et/ou d'aluminium.

13. Procédé selon les revendications 1 à 12, caractérisé en ce que les produits de réactions qui se produisent en premier lieu, sont soumis à un traitement final avec des agents de décoloration.
